## Europäisches Patentamt

## European Patent Office

## Office européen des brevets

(11) Veröffentlichungsnummer: **0 047 434**
**A1**

(12) **EUROPÄISCHE PATENTANMELDUNG**

(21) Anmeldenummer: **81106629.9**

(22) Anmeldetag: **26.08.81**

(51) Int. Cl.³: **G 01 N 33/00, G 01 N 27/56**

(30) Priorität: **05.09.80 DE 3033429**
**05.09.80 DE 3033428**

(43) Veröffentlichungstag der Anmeldung: **17.03.82**
**Patentblatt 82/11**

(84) Benannte Vertragsstaaten: **CH DE FR GB IT LI**

(71) Anmelder: **BROWN, BOVERI & CIE Aktiengesellschaft, Kallstadter Strasse 1, D-6800 Mannheim 31 (DE)**

(72) Erfinder: **Rohr, Franz-Josef, Dr., Forstweg 2, D-6941 Abtsteinach (DE)**

(74) Vertreter: **Kempe, Wolfgang, Dr. et al, c/o BROWN, BOVERI & CIE AG Kallstadter Strasse 1, D-6800 Mannheim-Käfertal (DE)**

(54) Elektrochemische Messvorrichtung.

(57) Die Erfindung bezieht sich auf eine Sauerstoffmeßvorrichtung insbesondere für Verbrennungsabgase, mit einem sauerstoffionenleitenden Festelektrolyten (16), der mit wenigstens zwei Elektroden (18 und 22) versehen ist, von denen eine erste Elektrode (18) als Meßelektrode eingesetzt und an einen Meßgaskanal (29) grenzt, der einen ersten Strömungskonstanthalter (64) aufweist und mit einer Meßgasquelle verbindbar ist. Beide Elektroden (18 und 22) sind unter Einschaltung eines Strommeßgerätes (106) mit einer elektrischen Gleichspannungsquelle (104) verbindbar. Erfindungsgemäß ist im Meßgaskanal (29) wenigstens ein weiterer auf einen festen Durchlaßwert eingestellter Strömungskonstanthalter (75 und 77) angeordnet, der dem ersten Strömungskonstanthalter (64) parallel schaltbar ist. Zusätzlich kann dem Meßgaskanal (29) eine Abzweigleitung (37) parallelgeschaltet werden. Die Abzweigleitung ist so angeordnet, daß sie mindestens den Bereich des Meßgaskanals (29) überbrückt, in dem sich die Meßelektrode (18) befindet. Über die Abzweigleitung (37) kann ein Teilstrom des Meßgases vor der Meßelektrode (18) abgezweigt werden. Vorzugsweise werden in den Meßgaskanal drei zueinander parallelgeschaltete Strömungskonstanthalter (64, 75 und 77) angeordnet. Die Strömungskonstanthalter (64, 75 und 77) können gleiche oder unterschiedlich große Durchtrittsquerschnitte aufweisen.

BROWN, BOVERI & CIE AKTIENGESELLSCHAFT
Mannheim                                06. Juli 1981
Mp.-Nr. 608/610/80                      ZPT/P1 - Kr/Sd

## Elektrochemische Meßvorrichtung

Die Erfindung bezieht sich auf eine elektrochemische Sauerstoffmeßvorrichtung, insbesondere für Verbrennungsabgase, mit
einem sauerstoffionenleitenden Festelektrolyten, der mit
wenigstens zwei Elektroden versehen ist, von denen eine erste
Elektrode als Meßelektrode eingesetzt und an einen Meßgaskanal grenzt, der einen ersten Strömungskonstanthalter aufweist und mit einer Meßgasquelle verbindbar ist, wobei beide
Elektroden unter Einschaltung eines Strommeßgerätes mit einer
elektrischen Spannungsquelle verbindbar sind.

Bei einer bekannten Sauerstoff-Meßvorrichtung dieser Art wird
während des Meßvorgangs der gesamte Sauerstoffanteil des Meß-

0047434

gasstroms elektrolytisch aus dem Meßgasstrom extrahiert, und
in Form von Sauerstoffionen durch den Elektrolyten zur
zweiten Elektrode transportiert und dort zu Sauerstoffmolekülen oxidiert. Der hierbei fließende und mit dem Strommeßgerät erfaßte Strom ist ein Maß für den Sauerstoffionentransport und somit für den Sauerstoffgehalt des Meßgasstroms. Um hierbei einen linearen oder zumindest weitgehend
linearen Zusammenhang zwischen dem elektrischen Strom und dem
Sauerstoffionentransport beziehungsweise dem Sauerstoffgehalt
des Meßgasstroms zu erzielen, ist es erforderlich, die Größe
des Meßgasstroms auf die Größe der ersten Elektrode abzustimmen, oder falls die Elektroden unterschiedliche Größen aufweisen sollten, den Meßgasstrom auf die Größe der kleineren
Elektrode abzustimmen. Der lineare Zusammenhang zwischen
Sauerstoffgehalt und elektrischem Strom gestattet die Eichung
der Sauerstoffmeßzelle mit Hilfe eines einzigen Eichpunktes
durchzuführen. Um eine quantitative Aussage über den Sauerstoffgehalt des Meßgasstroms machen zu können, ist es erforderlich, gegebenenfalls die Größe des Meßgasstroms zu erfassen oder eine Eichung durchzuführen und den Gasstrom während
des Meßvorgangs konstant zu halten.

Während des Betriebes wird bei bekannten Sauerstoff-Meßvorrichtungen dieser Art an die Elektroden eine elektrische
Gleichspannung gelegt und die als Kathode wirksame erste
Elektrode mit dem zu messenden Gasstrom beaufschlagt. Hierdurch wird der Sauerstoffanteil des Gasstromes elektrolytisch
aus dem Gasstrom extrahiert und in Form von Sauerstoffionen
durch den Festelektrolyten zur zweiten Elektrode transpor-

608/610/80                              3

tiert und dort wieder zu Sauerstoffmolekülen oxidiert. Der
bei diesem Transport fließende und mit dem Strommeßgerät
erfaßte elektrische Strom ist dann ein Maß für den Sauerstoffionentransport und somit für den Sauerstoffgehalt des
Gasstromes. Hierbei ist es jedoch erforderlich, den gesamten
Sauerstoff aus dem Gasstrom zu extrahieren, gegebenenfalls
die Größe des Gasstromes zu erfassen und hauptsächlich den
Gasstrom während des Meßvorgangs konstant zu halten.

Infolge von Temperaturänderungen der Meßvorrichtung, diese
muß nämlich auf einer Mindesttemperatur von ungefähr 400°C
gehalten werden, und/oder infolge von Alterung und/oder
Verschmutzung der Elektroden kann aus dem vorgegebenen
Gasstrom nicht mehr der vollständige Sauerstoffanteil extrahiert werden. Es ist daher am oberen Ende des Meßbereiches,
das heißt bei hohen Sauerstoffgehalten des Meßgases, der
lineare Zusammenhang zwischen dem Sauerstoffgehalt und dem
elektrischen Meßstrom nicht mehr vorhanden. Der ausnutzbare
lineare Meßbereich der Meßvorrichtung wird damit verkleinert.

Der Erfindung liegt die Aufgabe zugrunde eine elektrochemische Sauerstoff-Meßvorrichtung mit veränderbarem Meß- und/-
oder Empfindlichkeitsbereich so zu schaffen, daß eine exakte
Anpassung an die vorliegende Meßaufgabe möglich wird, wobei
die Veränderung des Meßbereichs und/oder der Empfindlichkeit
ohne wesentliche Betriebsunterbrechung durchführbar sein
soll. Die Meßvorrichtung soll ferner so ausgebildet sein, daß
Verkleinerungen des linearen Meßbereiches, die insbesondere
durch Alterung oder Verschmutzung der Elektroden hervorgeru-

608/610/80                    4

fen werden auf einfache Weise und zumindest ohne wesentliche Betriebsunterbrechungen ausgeglichen werden können und somit die weitgehende Wartungsfreiheit und Betriebssicherheit der Meßvorrichtung aufrechterhalten bleibt.

Die Lösung der Aufgabe ist dadurch gekennzeichnet, daß im Meßgaskanal wenigstens ein weiterer auf einen festen Durchlaßwert eingestellter zweiter Strömungskonstanthalter angeordnet und dem ersten Strömungskonstanthalter parallel geschaltet ist.

Bei einer Ausführungsform der Erfindung ist an den Meßgaskanal stromaufwärts der ersten Meßelektrode eine Abzweigleitung für die Abfuhr eines Meßgasteilstromes angeschlossen.

Eine weitere Lösung der oben stehenden Aufgabe ist dadurch gekennzeichnet, daß im Meßgaskanal wenigstens ein weiterer auf einen festen Durchlaßwert eingestellter zweiter Strömungskonstanthalter angeordnet und dem ersten Strömungskonstanthalter parallel geschaltet ist, oder daß an den Meßgaskanal stromaufwärts der ersten Meßelektrode eine Abzweigleitung für die Abfuhr eines Meßgasteilstromes angeschlossen ist. In den Weg des Meßgasstromes ist zunächst ein Strömungskontanthalter eingeschaltet, der einen Meßgasstrom von Nenngröße aufrecht erhält. Ein solcher Strömungskonstanthalter kann aus einer Pumpe mit einstellbarem konstanten Förderstrom bestehen. Einfacher und kostengünstiger ist jedoch eine Ausführungsform, bei der eine Verengung in den Meßgasstrom eingeschaltet ist, die bei Schallgeschwindigkeit

608/610/80 5

des Meßgases einen Meßgasstrom von Nenngröße durchläßt, wobei zwischen der Abströmseite und der Zuströmseite der Verengung ein die Schallgeschwindigkeit bewirkendes Druckverhältnis, nämlich das sogenannte kritische Druckverhältnis einstellbar ist. Um nun den Meßgasstrom vergrößern zu können, ist mindestens ein weiterer Strömungskonstanthalter vorgesehen, der auf einen festen Durchlaßwert eingestellt ist und dem ersten Strömungskonstanthalter parallel geschaltet werden kann.

Zur einfachen Verringerung des Meßgasstroms dient die Alternative, wonach ein Teilstrom des Meßgases stromaufwärts der ersten Meßelektrode durch eine Abzweigleitung abgeführt werden kann.

Bei der erfindungsgemäßen elektrochemischen Sauerstoff-Meßvorrichtung kann der Meßgasstrom auf konstante, von der Nenngröße abweichende Werte eingestellt werden. Da die Sauerstoff-Meßvorrichtung bei vorgegebener Elektrodengröße jeweils nur ein bestimmtes Sauerstoffvolumen in der Zeiteinheit aus dem Meßgasstrom extrahieren kann, ändert sich bei einer Veränderung des Meßgasstromes auf beliebige Werte die Steigung der Kennlinie und die Größe des Meßbereichs. Durch die erfindungsgemäße Lehre ist somit ein Weg gezeigt, wie auf überraschend einfache Weise eine Veränderung des Meßbereichs und/oder der Empfindlichkeit einer Sauerstoffmeßvorrichtung durchgeführt werden kann.

Hierbei wird vorteilhaft der Meßgasstrom zur Erweiterung des

608/610/80 6

Nenn-Meßbereichs und/oder zur Verringerung der Nenn-Empfindlichkeit vermindert, zur Verkleinerung des Meßbereichs
und/oder zur Steigerung der Nenn-Empfindlichkeit dagegen
vergrößert.

Zur Vereinfachung des gesamten Aufbaus ist vorzugsweise der
zweite Strömungskonstanthalter identisch mit dem ersten
Strömungskonstanthalter ausgeführt. Er weist eine Verengung
auf, die einen Durchtrittsquerschnitt besitzt, welcher
insbesondere bei Schallgeschwindigkeit des Meßgases jeden
gewünschten zusätzlichen Meßgasstrom hindurchläßt. Zwischen
der Abströmseite und der Zuströmseite der Verengung des
zweiten Strömungskonstanthalters ist ein Druckverhältnis
einstellbar, das in der Verengung die Schallgeschwindigkeit
des Gasstromes bewirkt. Die Verengung besteht vorzugsweise
aus einer Düse oder einer Blende. Um den Meßgasstrom
vergrößern zu können, ist bei einer Ausführungsform der
Erfindung ein dritter Strömungskonstanthalter vorgesehen, der
auf einen festen Durchlaßwert eingestellt ist und der dem
ersten und dem zweiten Strömungskonstanthalter parallel
geschaltet werden kann.

Für den Fall, daß eine Verringerung des Meßgasstromes
gewünscht wird, ist in vorteilhafter Weise in die Abzweigleitung eine vorzugsweise einstellbare Drosselvorrichtung
eingebaut.

Steht das zur Sauerstoff-Meßvorrichtung strömende Meßgas
unter Überdruck und ist der erste Strömungskonstanthalter vor der Abzweigleitung in den Meßkanal eingebaut, so ist es am einfachsten die Abzweigleitung in den Außenraum münden zu lassen.

Befindet sich der Strömungskonstanthalter dagegen stromabwärts des Meßelektrode und/oder weist das Meßgas Unterdruck gegenüber dem Aussenraum auf, so ist es empfehlenswert, das zweite Ende der Abzweigleitung an den Meßgaskanal stromab der Meßelektrode vor dem Strömungskonstanthalter anzuschließen. In diesem Fall bildet die Abzweigleitung einen Bypass, durch den ein Teil des Meßgasstromes die Sauerstoff-Meßzelle umgeht.

Die Einstellung des Meßgasstroms auf verschiedene konstante Werte kann wie bereits dargelegt durch eine in der Abzweigleitung angeordnete einstellbare Drosselvorrichtung erfolgen. Einfacher ist es jedoch, die Abzweigleitung in mehrere parallel geschaltete wahlweise einschaltbare Teilleitungen aufzuteilen.

Hierdurch kann jede Teilleitung auf einen bestimmten Strömungswiderstand eingestellt werden und durch die beliebige Kombination dieser Teilleitungen der gewünschte Meßgas-Teilstrom auf einfache Weise gewählt werden. Dies geschieht vorzugsweise durch Absperrorgane, die in die Teilleitungen eingebaut sind und in entsprechender Weise geschlossen beziehungsweise geöffnet werden.

Bei einer Sauerstoff-Meßvorrichtung, deren Elektrolyt den Meßgaskanal von einem vorzugsweise an die zweite Elektrode

608/610/80                    8

grenzenden Elektrodenraum trennt, ist vorzugsweise die
Abzweigleitung von einer Durchtrittsöffnung des Elektrolyten
und dem Elektrodenraum gebildet. Hierbei ist vorzugsweise die
Durchtrittsöffnung als Drosselstelle ausgebildet, welche die
Größe des Meßgas-Teilstromes hauptsächlich bestimmt.

Eine andere bevorzugte Weiterbildung der Erfindung besteht
darin, daß ein rohrförmiger, an einem Ende geschlossener
Elektrolyt vorgesehen ist. Dieser wird von einem ringförmigen
Meßkanal umgeben. Im Inneren des Elektrolyten ist ein Elektrodenraum vorgesehen, der mit einem axial verlaufenden
Heizstab versehen ist, wobei zwischen dem Heizstab und dem
Elektrolyten ein Ringraum gebildet ist. Bei dieser
Ausführungsform wird die Abzweigleitung von mindestens einer
Durchtrittsöffnung am geschlossenen Ende des Elektrolyten und
dem im Inneren des Elektrolyten befindlichen Elektrodenraums
gebildet. Der Querschnitt der Durchtrittsöffnung kann durch
den axial verschiebbaren Heizstab verändert werden. Der
Heizstab dient somit als Absperrorgan, mit dem der Querschnitt der Durchtrittsöffnung einstellbar beziehungsweise
absperrbar ist. Zur Erzielung einer günstigen Drosselcharakteristik und für eine sichere Absperrung des Durchtrittsquerschnittes ist es empfehlenswert, daß die Durchtrittsöffnung
sich in Strömungsrichtung kegelförmig erweitert und die
Spitze des Heizstabes einen entsprechenden Gegenkegel aufweist.

Wird der an die zweite Elektrode angrenzende Elektrodenraum
als Abzweigleitung benutzt, so ist vorteilhaft die elek-

608/610/80                              9

trische Spannungsquelle über einen Polumschalter in den elektrischen Stromkreis einschaltbar. Durch die Betätigung dieses
Umschalters wird die Polung der Elektroden umgeschaltet und
somit wechselweise die erste oder zweite Elektrode als
Meßelektrode eingesetzt. Fließen hierbei in den Raum, der an
die erste Elektrode angrenzt und in den im Elektrolyten
befindlichen Elektrodenraum verschieden große Gasströme, so
kann durch die Umschaltung der Polarität der Elektroden
jeweils der für den jeweils vorliegenden Anwendungsfall
günstigste Gasstrom als Meßgasstrom benutzt werden. Hierdurch
ergibt sich eine einfache Veränderungsmöglichkeit des Meßbereichs und/oder der Meßempfindlichkeit der elektrochemischen
Meßvorrichtung ohne Eingriff in den Fluß des Meßgasstroms.

Zur Vermeidung einer durch Alterung und/oder Verschmutzung
der Elektroden bedingten Verkleinerung des linearen Meßbereiches sind erfindungsgemäß im Weg des Gasstroms drei  als
Strömungskonstanthalter dienende Verengungen angeordnet. Die
Durchtrittsquerschnitte dieser Verengungen sind so gewählt,
daß bei einer Parallelschaltung dieser Verengungen ein
ausreichender maximaler Gasstrom durchgelassen wird. Bei
einer Ausführungsform der Erfindung ist die Größe der ersten
Verengung so gewählt, daß die Größe des durch sie hindurchtretenden Meßgasstroms auf die Größe der ersten Elektrode im
Neuzustand der Meßzelle abgestimmt ist.

In vorteilhafter Weise können die drei Verengungen wahlweise
einzeln, in Parallelschaltung gemeinsam oder beliebig kombiniert in den Weg des Gasstromes eingeschaltet werden.

608/610/80                              10

Bei dieser Ausführungsform der Sauerstoff-Meßvorrichtung wird der für die Beaufschlagung der ersten Elektrode erforderliche konstante Gasstrom, dessen Größe auf die Größe dieser Elektrode abgestimmt ist, nicht mehr in einer einzigen Verengung (Blende oder Düse) erzeugt. Wird eine Sauerstoff-Meßvorrichtung verwendet, die Elektroden unterschiedlicher Größe aufweist, so wird die Größe des Gasstromes auf die kleinere Elektrode abgestimmt. Die Abstimmung erfolgt auf die Elektrode im Neuzustand. Bei der hier beschriebenen Ausführungsform der Erfindung werden vorzugsweise drei Verengungen beziehungsweise Strömungskonstanthalter in den Weg des Gasstromes eingefügt und wahlweise in beliebiger Kombination in Betrieb genommen. Im Neuzustand der Meßzelle, in dem die Meßelektrode mit dem vollen Gasstrom beaufschlagt werden kann, sind sämtliche Verengungen in Betrieb. Es besteht die Möglichkeit, daß an der Meßzelle während des Betriebs Verschmutzungen, Alterungen und/oder Temperaturänderungen auftreten, welche die Wirksamkeit der Elektroden und/oder des Festelektrolyten bezüglich der Extration des Sauerstoffanteils beziehungsweise der Sauerstoffionenleitfähigkeit verändern. Dadurch kommt es zu einer Verkleinerung des linearen Meßbereichs. Werden solche Erscheinungen bei der Meßzelle festgestellt, so wird nur noch ein Teil der Verengungen in Betrieb genommen, das heißt der Durchfluß des Meßgasstromes wird auf einen kleineren konstanten Wert verringert. Hierdurch wird die für eine Linearität der Meßanzeige erforderliche Abstimmung zwischen der Größe der Meßelektrode und der Größe des Meßgasstromes erzielt.

608/610/80                           11

Durch noch weiteres Verringern des Meßgasstromes kann hierbei zusätzlich eine Erweiterung des linearen Meßbereiches über die Größe des anfänglichen Nennbereiches hinauserreicht werden.

Die Erfindung wird nachfolgend anhand von Zeichnungen erläutert. Weitere Merkmale der Erfindung gehen aus der nachfolgenden Beschreibung der Ausführungsbeispiele im Zusammenhang mit den schematischen Zeichnungen hervor.


Es zeigen:

Fig.   1   einen Längsschnitt durch eine Sauerstoff-Meßvorrichtung gemäß der Erfindung mit einer Abzweigleitung,

Fig.   2   einen Längsschnitt durch eine Saugpumpe für Handbetrieb, die als Ausführungsvariante an den Pufferraum der Fig. 1 anschließbar ist,

Fig.   3   einen Längsschnitt durch einen als Düse ausgebildeten Strömungkonstanthalter,

Fig.   4   eine Ausführungsvariante der Fig. 1,

Fig.   5   eine weitere Ausführungsvariante des Gegenstandes der Erfindung, wobei mehrere Strömungkonstanthalter in Form von Düsen parallel geschaltet sind,

608/610/80                                 12

Fig. 6 u. die Kennlinien der Sauerstoff-Meßvorrichtungen
Fig. 7    gemäß der Fig. 1 bis 5.


Gleiche Teile sind in den einzelnen Figuren mit den gleichen
Bezugszeichen versehen. Ferner sind in den einzelnen Figuren
wiederkehrende  Einzelteile nur insoweit mit Bezugszeichen
versehen, als dies für das Verständnis erforderlich ist.

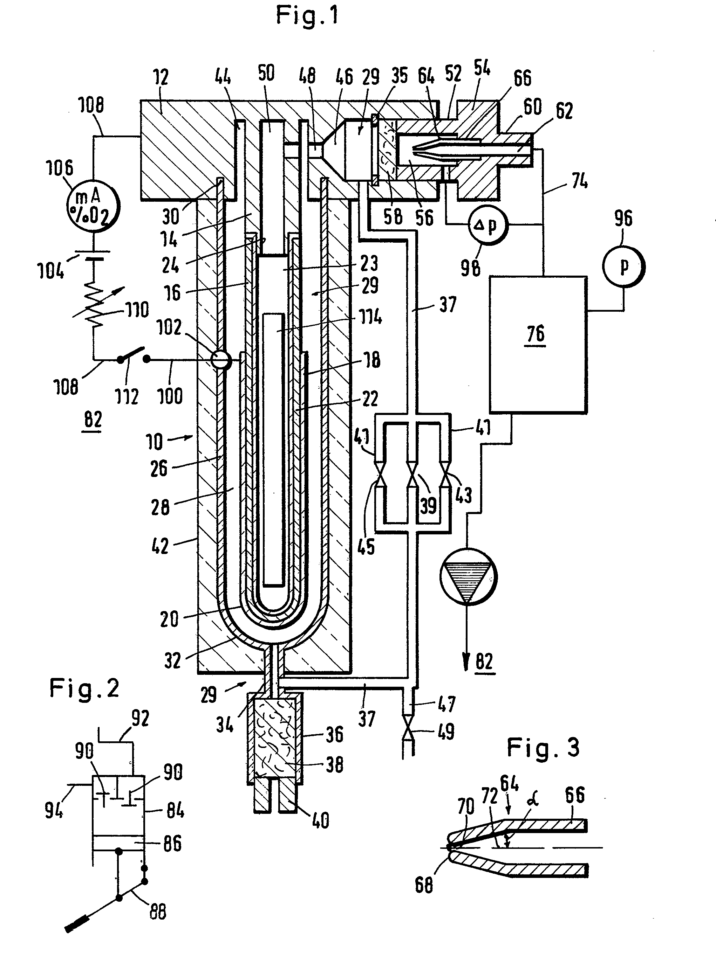

Gemäß Fig. 1 weist die elektroschemische Sauerstoff-Meßvorrichtung eine Meßzelle 10 mit einem etwa quadratförmigen
Grundkörper 12 auf, der zum Beispiel aus Metall gefertigt
ist. Aus der einen Grundfläche des Grundkörpers 12 ragt ein
kreisringzylindrischer Vorsprung 14, an dessem freien Ende
ein Festelektrolytrohr 16 befestigt ist. Das Festelektrolytrohr 16 ist auf seiner Aussenseite mit der ersten Elektrode
18 versehen, die von der verschlossenen Spitze 20 aus gesehen
etwa zwei Drittel der Festelektrolytrohrlänge bedeckt. Auf
der Innenseite des Festelektrolytrohres 16 ist die zweite
Elektrode 22 vorgesehen, die sich von der Spitze 20 bis zur
Befestigungsstelle des Festelektrolytrohres 16 am Vorsprung
14 erstreckt und in elektrisch leitendem Kontakt mit dem
Vorsprung 14 steht. Dies ist im vorliegenden Ausführungsbeispiel dadurch erreicht, daß die zweite Elektrode 22 mit dem
Zentriervorsprung 24 in Berührung steht. Die zweite Elektrode
22 grenzt an den Elektrodenraum 23. Die beiden Elektroden 18
und 22 bestehen aus einem keramischen oder metallischen
Material, durch welches der Sauerstoff ungehindert hindurchtreten kann. Vorzugsweise werden die Elektroden aus einem
porösen Material hergestellt. Hierfür geeignete Werkstoffe
sind zum Beispiel Silber oder Platin. Als Werkstoff für das
sauerstoffionenleitende Festelektrolytrohr kann Zirkoniumoxid
verwendet werden, das mit Kalciumoxid dotiert ist.

Das mit den beiden Elektroden 18 und 22 versehene Festelektrolytrohr 16 ist von einem Hüllrohr 26 umgeben, so daß
zwischen der ersten Elektrode 18 und dem Hüllrohr 26 ein Raum
28 mit kreisringförmigem Querschnitt gebildet ist, wobei die
lichte Weite dieses Raumes 28 in radialer Richtung etwa 0,3
bis 2 mm, vorzugsweise etwa 0,5 mm beträgt. Wegen der Übersichtlichkeit ist in den Figuren diese Weite größer dargestellt. Das Hüllrohr 26 ist in eine kreisringförmige Aussparung 30 des Grundkörpers 12 eingesteckt und befestigt. Das
zweite Ende des Hüllrohres ist kuppenförmig verschlossen,
wobei der Abstand der Verschlußkuppe 32 zur ersten Elektrode
18 der ebenfalls kuppenförmigen Spitze 20 etwa gleich ist dem
Abstand zwischen dem zylindrischen Bereich des Hüllrohres 26
und der ersten Elektrode 18. Der Raum 28 ist Bestandteil des
Meßgaskanals 29.

An der Spitze der Verschlußkuppe 32 ist ein Rohrstutzen 34
vorgesehen, der in einer Erweiterung 36 ein Gasfilter 38
aufweist, das zum Beispiel aus Watte oder aus einem porösen
Keramikkörper besteht. Schließlich ist die Erweiterung 36 mit
einem axial durchbohrten Nippel 40 verschlossen, durch
welchen das zu messende Gas zugeführt wird. Das gesamte
Hüllrohr 26 ist schließlich noch mit einem Isoliermantel 42 umgeben, der sich bis zum Grundkörper 12 erstreckt.

Als Material für den Isoliermantel kann zum Beispiel Mineralwolle eingesetzt werden.

Der am Grundkörper 12 angeordnete ringförmige Vorsprung 14
ist von einem im Grundkörper verlaufenden konzentrischen
Ringspalt 44 umgeben, der einerseits in den Raum 28 mündet,
und der andererseits an einen quer zur Längsachse der
Meßzelle verlaufenden, im Querschnitt kreisförmigen Raum 46
angeschlossen ist. Die quer zur Längsachse verlaufende
Anschlußbohrung 48 ist hierbei in Richtung zur Längsachse
weitergeführt, so daß auch der Innenraum 50 des Vorsprungs
14, welcher mit dem Innenraum des Festelektrolyten 16,
nämlich dem Elektrodenraum 23, eine Einheit bildet, an den
Raum 46 angeschlossen ist. Der Raum 46, der vorzugsweise als
Bohrung ausgebildet ist, weist wenigstens ein Gewinde auf. In
dieses ist ein Einsatz 52 mit Hilfe eines am Einsatz vorgesehenen Kopfes 54 eingeschraubt. Der in den Grundkörper 12
ragende Bereich des Einsatzes 52 ist kreisringförmig ausgebildet und klemmt ein scheibenförmiges Filter 58 zwischen
sich und einem Haltering 35 ein. Eine Verjüngung bildet
hierbei den Übergang des Raumes 46 zur Anschlußbohrung 48.
Das Filter 58 ist bezüglich seines Materials identisch mit
dem Gasfilter 38.

Am Kopf 54 ist ein nach außen ragender Anschlußnippel 60
zentrisch angeordnet, der von einem zentrisch verlaufenden
und zur Ausnehmung 56 führenden Kanal 62 durchdrungen ist. Im

608/610/80                                    15

Bereich seiner inneren Ausmündung weist der Kanal 62 eine Stufe auf, in welcher die Düse 64 mit ihrem zylindrischen Bereich 66 befestigt ist. Die Düse 64 ragt frei in die Ausnehmung 56 hinein. Die Düse 64 bildet den ersten Strömungskonstanthalter. Der Aufbau der Düse 64 ist am besten anhand von Figur 3 zu sehen. Hier ist die Düse 64 alleine und in größerem Maßstab dargestellt. Wie Figur 3 zeigt ist der Einlauf 68 der Düse 64 abgerundet und führt zum engeren Durchtrittsquerschnitt 70, an den sich eine kegelförmige Erweiterung 72 anschließt, wobei der Öffnungswinkel $\alpha$ der Erweiterung etwa 8° bis 18° , vorzugsweise 10° bis 15° beträgt. An die Erweiterung 72 schließt sich der zylindrische Bereich 66 an.

Die engste Stelle des Durchtrittsquerschnittes 70 ist nun so gewählt, daß bei einer Durchströmung dieser Stelle mit Schallgeschwindigkeit eine für Nennbetrieb ausreichende Gasmenge durchtritt. Als Richtwert für den Durchtrittsquerschnitt 70 kann im vorliegenden Ausführungsbeispiel, bei dem der Raum 28 einen Querschnitt von 10 bis 20 $mm^2$ aufweist, ein Wert von etwa 0,8 bis 20 x $10^{-4}$ $mm^2$ gelten. Die Gesamtlänge der Düse beträgt etwa 15 bis 30 mm. Die Länge der Erweiterung 72 liegt zwischen 5 und 10 mm bei einer Lichtweite des zylindrischen Bereiches 66 von etwa 5 bis 8 mm. Als bevorzugtes Material für die Düse dient Glas, auch mit Laserstrahlen durchbohrte Saphire sind empfehlenswert. Der gasführende Querschnitt der Düse ist kreisförmig, das Gleiche gilt auch für die Blende 120. Die Fläche der ersten Elektrode 18 beträgt ungefähr 5 bis 50 $cm^2$ ,vorzugsweise 10 bis   30

$cm.^2$.

Wie anhand von Figur 1 zu sehen ist, verbindet die Düse 64 die Ausnehmung 56, welche die Zuströmseite des Gases bildet, mit dem Kanal 62, welcher die Abströmseite der Düse 64 darstellt. An den Anschlußnippel 60 ist über eine Anschlußleitung 74 ein Pufferraum 76 angeschlossen, der über eine weitere Leitung 78 mit einer Saugvorrichtung 80, zum Beispiel in Form einer Gaspumpe, verbunden ist. Der Auslaß der Gaspumpe mündet in den Außenraum 82. Enthält das Meßgas giftige Bestandteile, so mündet der Auslaß der Gaspumpe in einen Auffangraum.

An den Rohrstutzen 34, welcher den Meßgaskanal 29 mitbildet und der stromaufwärts der ersten Elektrode 18 angeordnet ist, ist die Abzweigleitung 37 angeschlossen. Diese umgeht den Raum 28 und mündet in den Raum 46, der den Meßgaskanal ebenfalls mitbildet und stromabwärts der ersten Elektrode angeordnet ist. In der Abzweigleitung 37 ist ein Drossel- beziehungsweise Absperrorgan 39 angeordnet. Dieses ist durch zwei Teilleitungen 41 überbrückt, welche ebenfalls Drossel- beziehungsweise Absperrorgane 43 beziehungsweise 45 enthalten. Schließlich ist noch die Abzweigleitung 37 mit einer Ausströmleitung 47 versehen, die ebenfalls ein Drossel- beziehungsweise ein Absperrorgan 49 enthält. Die Ausströmleitung 47 ist dazu vorgesehen, einen Teilstrom des Meßgases unmittelbar in den Aussenraum 82 abzuleiten.

In Figur 2 ist eine Ausführungsvariante bezüglich der Saug-

vorrichtung dargestellt, die anstatt der Saugvorrichtung 80 an den Pufferraum 76 angeschlossen werden kann. Die Saugvorrichtung gemäß Figur 2 besteht aus einer Handpumpe 84, deren Kolben 86 durch einen Pumpenhebel 88 betätigt werden kann, so daß über die angedeuteten Ventilen 90 Gas aus dem Pufferraum 76 über die Leitung 92 angesaugt und durch den Auslaß 94 in den Außenraum ausgestoßen werden kann.

Zur Erfassung der Druckverhältnisse des Gasstromes ist im Pufferraum 76 ein Manometer 96 angeordnet. Gegebenenfalls kann auch ein Differenzmanometer 98 zwischen die Ausnehmung 56 und die Anschlußleitung 74 oder den Kanal 62 eingeschaltet sein.

Um an die Elektroden 18 und 22 die erforderliche Meßspannung geben zu können, führt von der ersten Elektrode 18 eine elektrische Leitung 100 radial in den Außenraum 82, wobei an der Durchdringungsstelle des Hüllrohres 26 eine Isolierperle 102 eingefügt ist. Die zweite Elektrode 22 ist über den Zentriervorsprung 24 und den Vorsprung 14 mit dem Grundkörper 12 elektrisch leitend verbunden. Der Grundkörper 12 bildet zusammen mit der elektrischen Leitung 100 die elektrischen Anschlüsse der Elektroden 18 und 22. Diesen Anschlüssen wird die für die Messung erforderliche Spannung über elektrische Leitungen 108 von einer Gleichspannungsquelle 104, zum Beispiel in Form einer elektrischen Batterie, zugeführt. In die elektrische Verbindung zwischen der ersten und zweiten Elektrode 18 und 22 ist zusätzlich ein Strommeßgerät 106 zum Beispiel ein Milliamperemeter, ein Regelwiderstand       110

0047434

608/610/80                    18

und ein elektrischer Schalter 112 eingefügt.

Im Elektrodenraum 23 innerhalb des Festelektrolyten 16 ist noch eine stabförmige, vorzugsweise elektrische Heizung 114 angeordnet. Diese hat die Aufgabe das Festelektrolytrohr 16 auf Betriebstemperatur zu bringen. Die zur Heizung 114 führenden elektrischen Anschlußleitungen sind in Figur 1 nicht dargestellt. Die Mindestbetriebstemperatur beträgt ca. $400^{\circ}$ C.

Bevor mit der Sauerstoffmeßvorrichtung Messungen durchgeführt werden können, ist eine Ersteinstellung beziehungsweise Eichung erforderlich, die am besten gleich bei der Herstellung der Meßvorrichtung durchgeführt wird. Hierzu ist es am einfachsten, ein Gas zu benutzen, das in chemischer und physikalischer Hinsicht dem zu messenden Gas ähnlich ist. Soll die Meßvorrichtung zur Bestimmung des Sauerstoffgehaltes in Abgasen eingesetzt werden, so kann für die Einrichtung Luft benutzt werden. Das kritische Druckverhältnis für Luft als zweiatomiges Gas beträgt hierbei 0,528. Der Nennmeßbereich soll hierbei 21 Volumenprozent Sauerstoff umfassen.

Für die Ersteinstellung wird zunächst die Heizung 114 und dann die Saugvorrichtung 80 in Betrieb genommen und deren Saugleistung so eingestellt, daß das Druckverhältnis zwischen der Abströmseite 62 und der Zuströmseite 56 der Düse 64 gleich dem kritischen Druckverhältnis oder vorzugsweise kleiner ist. Die Einstellung geschieht hierbei mit Hilfe des Differenzdruckmanometers 98. Ist der Druck des zugeführten Eichgases bekannt, zum Beispiel beim Ansaugen von Umgebungsluft, so genügt auch die Messung des Druckes auf der Abströmseite der Düse, zum Beispiel die Messung des Druckes im
Pufferraum 76 für die Bestimmung des Druckverhältnisses. Ist
die Ersteinstellung der Saugvorrichtung 80 einmal auf diese
Weise durchgeführt worden, so ist eine weitere laufende
Überwachung des Druckverhältnisses nicht erforderlich, wenn
diese Einstellung beibehalten wird. Ein Nenngasstrom von
gleichbleibender Größe ist somit auch im Langzeitbetrieb
gewährleistet. Für den Fall, daß das zu messende Gas der
Meßvorrichtung mit Überdruck zugeführt wird, sind eine
Saugvorrichtung 80 und gegebenenfalls ein Pufferraum 76 nicht
erforderlich, wenn die Abströmseite 62 der Düse 64 auf
niedrigerem Druck liegt, zum Beispiel durch Verbindung mit
dem Außenraum, und sich das gewünschte Druckverhältnis somit
einstellt.

Ist nun auf vorgeschriebene Weise die Ersteinstellung durchgeführt worden und der Festelektrolyt auf Betriebstemperatur
gebracht, so wird der Schalter 112 geschlossen und an die
Elektroden 18 und 22 eine Gleichspannung von etwa 1 Volt
gelegt. Die Regulierung dieser Spannung kann durch den
Regelwiderstand 110 erfolgen. Der Anzeige des Strommeßgerätes
106 wird jetzt ein Sauerstoffgehalt von 21 Volumenprozent
zugeordnet. Da beim vorliegenden Meßprinzip ein linearer
Zusammenhang zwischen dem Sauerstoffgehalt des zu messenden
Gases und dem im Meßkreis fließenden Strom besteht, genügt
ein einziger Meßpunkt für die Eichung.

608/610/80                              20

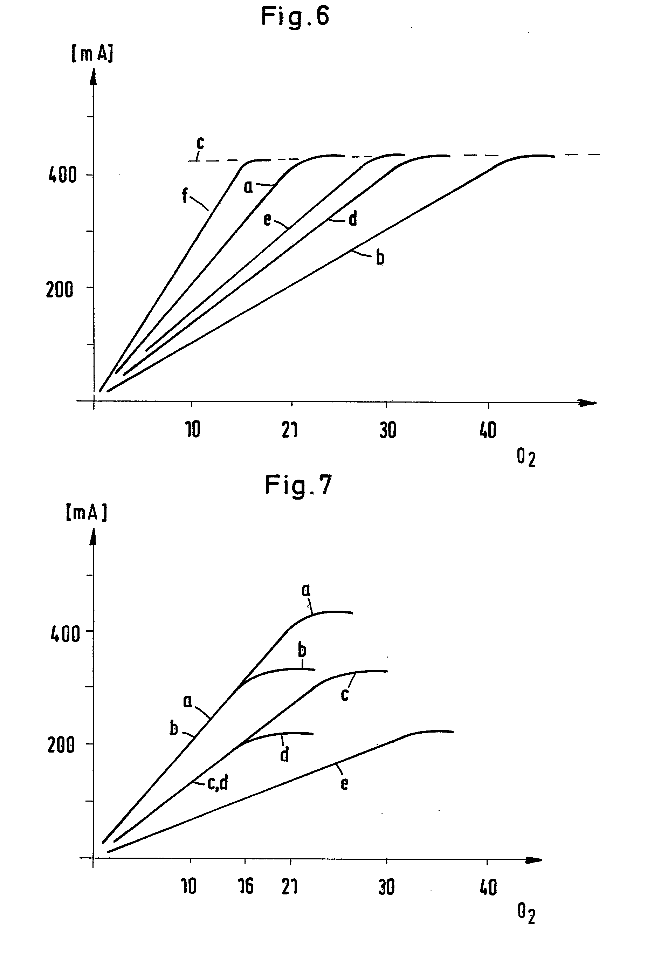

Bei der vorgeschriebenen Ersteinstellung beziehungsweise Eichung sind die Drossel- beziehungsweise Absperrorgane 39, 43, 45, 49 geschlossen, so daß der gesamte Meßgasstrom an der ersten Elektrode 18 vorbeigeführt wird und die Meßvorrichtung daher mit dem Nennmeßbereich 21 Volumenprozent und mit der Nennemfpindlichkeit arbeitet. Die Kennlinie dieser auf Nenndaten ausgelegten Meßzelle ist in Figur 6 dargestellt und mit a bezeichnet. Auf die gleiche Weise werden weitere Eichungen der Meßvorrichtung vorgenommen, bei denen jeweils eines der Drossel- beziehungsweise Absperrorgane 39, 43, 45 geschlossen ist. In diesem Fall wird jetzt ein Teilstrom vom Meßgasstrom abgezweigt und umgeht den Raum 28, so daß dort der Meßgasstrom verringert ist, während der Strömungskonstanthalter 64 mit gleichbleibendem konstantem Durchsatz arbeitet. Durch mehr oder weniger weites Öffnen der Drossel- beziehungsweise Absperrorgane kann der Durchfluß auf beliebige konstante Werte einreguliert werden. Auch kann der Strömungswiderstand beziehungsweise Gasdurchfluß ohne Drosselorgane durch entsprechende Ausbildung der Leitungsquerschnitte beziehungsweise Einzelwiderstände wie Umlenkungen festgelegt werden.

Im vorliegenden Ausführungsbeispiel soll der Strömungswiderstand der Abzweigleitung 37 beziehungsweise der Teilleitung 41 so ausgelegt sein, daß bei einer Öffnung des Drossel- beziehungsweise Absperrorgans 43 der Meßgasstrom in zwei gleiche Hälfte aufgteilt wird, von denen die eine durch den Raum 28 strömt und die Meßelektrode 18 beaufschlagt, wogegen die zweite Hälfte die Meßvorrichtung umgeht. Bei der Eichung

608/610/80                    21

der Meßvorrichtung mit Luft von 21 Volumenprozent Sauerstoff ergibt sich jetzt eine Kennlinie, die in Figur 6 mit b bezeichnet ist. Die Steigung dieser Kennlinie ist entsprechend der Verringerung des Meßgasstroms ebenfalls auf die Hälfte verringert und somit der lineare Meßbereich erheblich vergrößert. Der lineare Meßbereich erstreckt sich vom Nullpunkt bis etwa zu einem Sättigungszustand, in welchem die Leistungsgrenze der Meßelektrode 18 erreicht ist und in dem die Kennlinie einen etwa horizontalen Verlauf aufweist, der durch eine horizontale Grenzgerade c bezeichnet ist.

Beim alleinigen Öffnen des Drossel- beziehungsweise Absperrorgans 39 soll ein Drittel des Meßgasstroms über die Abzweigleitung 37 geführt werden, so daß die Kennlinie dementsprechend eine Steigung aufweist, die um ein Drittel gegenüber der Nenngröße verringert ist. Dieser Kennlinienverlauf ist in Figur 6 mit d bezeichnet.

Schließlich soll bei der alleinigen Öffnung des Drossel- beziehungsweise Absperrorgans 45 ein Viertel des Nenngasstromes über die Abzweigleitung 37 fließen, so daß sich bei der Eichung der Meßvorrichtung mit Luft eine Kennlinie e einstellt, deren Steigung gegenüber der Nennsteigung der Kennlinie a um ein Viertel verkleinert und der Meßbereich entsprechend vergrößert ist. Selbstverständlich lassen sich durch kombiniertes Öffnen der Drossel beziehungsweise Absperrorgane 39, 43, 45 verschieden große Meßgasteilströme einstellen und somit entsprechende Neigungen beziehungsweise Empfindlichkeiten und Meßbereiche der elektrochemischen

608/610/80                         22

Sauerstoffmeßvorrichtung erreichen.

Eine Ausführungsform, bei der die Abzweigleitung 37 die erste
Elektrode 18 umgeht, wird man dann anwenden, wenn der
Strömungskonstanthalter stromabwärts der ersten Elektrode 18
angeordnet ist und/oder wenn das ganze System unter Unterdruck steht. Steht dagegen der Meßgasstrom beim Eintritt in
die Meßzelle unter Überdruck und ist der Strömungskonstanthalterstrom stromaufwärts der Abzweigleitung 37 angeordnet,
so ist es gegebenenfalls nicht erforderlich, die
Abzweigleitung 37 wieder in den Meßgaskanal stromabwärts der
ersten Elektrode 18 münden zu lassen. In diesem Fall genügt
es, wenn der vorgesehene Meßgasteilstrom durch Öffnungen des
Drossel-, beziehungsweise Absperrorgans 49 ins Freie
abgeblasen wird. Es leuchtet ein, daß hierbei mehrere dieser
Drossel- beziehungsweise Absperrorgane vorgesehen sein
können, deren Durchfluß jeweils auf einen anderen Wert
eingestellt ist, um so verschiedene Neigungen der Kennlinie
zu erreichen.

Mit dieser Ausführungsform der Sauerstoffmeßvorrichtung
können auch Alterungen und/oder Temperaturänderungen des
Festelektrolyten und/oder Verschmutzungen der Elektroden
ausgeglichen werden, die zu einer Verkleinerung des linearen
oder zumindest weitgehend linearen Nenn-Meßbereichs führen.
Treten solche Verkleinerungen des Nenn-Meßbereichs auf, so
kann durch die oben beschriebene Verringerung des Meßgasstroms der Meßberreich erweitert oder der ursprüngliche
Nenn-Meßbereich wieder erreicht werden, wobei allerdings eine

608/610/80                          23

Verringerung der Empfindlichkeit in Kauf genommen werden muß. Auf der anderen Seite kann bei dieser erfindungsgemäßen Sauerstoff-Meßvorrichtung die Empfindlichkeit einer elektrochemischen Meßzelle eingestellt und auf den jeweiligen Einsatzfall der Meßvorrichtung abgestimmt werden.

Soll mit der Meßvorrichtung jetzt der Sauerstoffgehalt von Verbrennungsabgasen im Nennbereich gemessen werden, so ist die Meßvorrichtung mit Hilfe des Nippels 40 und gegebenenfalls unter Zwischenschaltung eines Gaskühlers mit der Abgasleitung zu verbinden. Die Saugvorrichtung 80 saugt jetzt einen konstanten Gasstrom durch den Meßgaskanal, der folgende Teile umfaßt: ein Gasfilter 38, den Raum 28, die Anschlußbohrung 48, den Raum 46, das Filter 58, die Ausnehmung 56, welche gleichbedeutend ist mit der Zuströmseite der Düse 64, die Düse 64, den Kanal 62, welcher gleichbedeutend ist mit der Abströmseite der Düse 64, den Pufferraum 76 und die Saugvorrichtung 80. Beim Vorbeiströmen des Gasstromes an der ersten Elektrode 18 wird der im Gasstrom enthaltene Sauerstoff extrahiert, in Form von Ionen durch den Festelektrolyten 16 geleitet und an der zweiten Elektrode 22 wieder zu Sauerstoff oxidiert und in den Innenraum des Festelektrolytrohres 16 abgegeben. Der Sauerstoff strömt dann durch die im Vorsprung 14 vorgesehene radiale Anschlußbohrung 48 ab und wird dem Gasstrom wieder beigemischt. Der von der Gleichspannungsquelle 104 bei geschlossenem Schalter 112 über die Elektroden 18 und 22 fließende Strom wird hierbei vom Strommeßgerät 106 erfaßt. Dieser Strom ist ein Maß für den Sauerstoffgehalt des Gases, gegebenenfalls erfolgt die

608/610/80                            24

Anzeige des Strom-Meßgerätes unmittelbar in Volumenprozent Sauerstoff. Im vorliegenden Fall sind die Drossel- beziehungsweise Absperrorgane 39, 43, 45 und 47 geschlossen. Die hierzu gehörige Kennlinie der Meßzelle besitzt den Verlauf gemäß a der Figur 6. Durch Öffnen der einzelnen Drossel- beziehungsweise Absperrorgane 43, 39 und 45 kann dann, wie vorbeschrieben der Nenn-Meßbereich und/oder die Nennempfindlichkeit verändert und an den vorgegebenen Meßfall angepaßt werden.

In den meisten Fällen wird die Saugvorrichtung 80 als elektrische Saugpumpe ausgebildet sein. Für Kurzzeitmessungen, zum Beispiel für Betriebsüberwachungen, ist es jedoch empfehlenswert, anstelle der Saugvorrichtung 80 eine Handpumpe 84 einzusetzten. Dieser Fall ist in Figur 2 dargestellt. Mit Hilfe der Handpumpe 84, die an den Pufferraum 76 angeschlossen ist, wird im Pufferraum 76 ein für die Erzeugung des kritischen Druckverhältnisses ausreichender Unterdruck erzeugt und dadurch die Wirkung des Pufferraumes 76 konstant gehalten. Wird hierbei der für die Erzeugung des kritischen Druckverhältnisses erforderliche Unterdruck im Pufferraum 76 stark unterschritten, so ist ein intermittierender Betrieb der Handpumpe 84 möglich, denn in den Betriebspausen der Pumpe sichert der Unterdruck des Pufferraumes 76 den für die Messung erforderlichen konstanten Gasfluß.

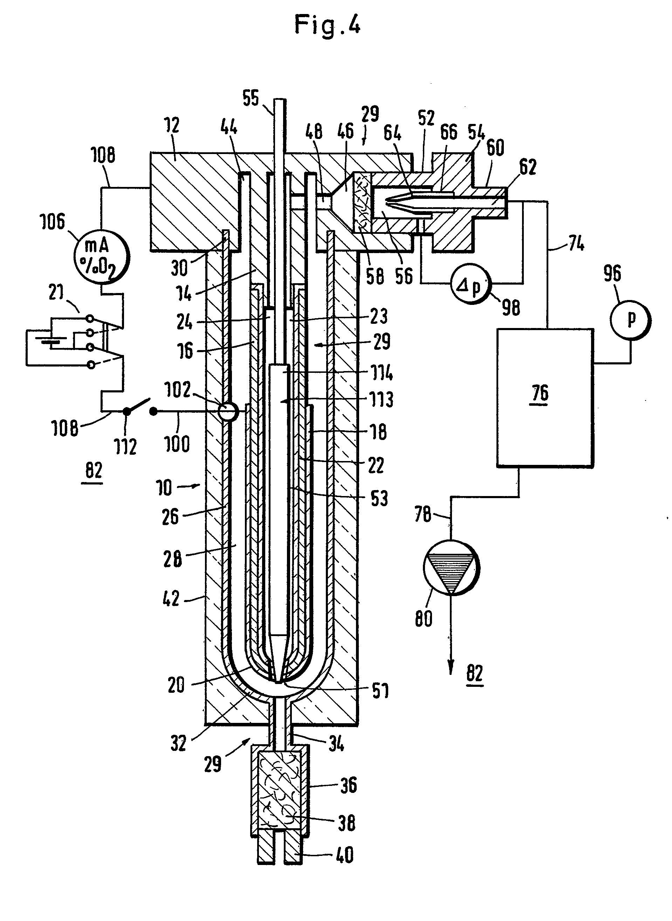

In Figur 4 ist eine Ausführungsvariante des Gegenstands der Figur 1 dargestellt. Während in Figur 1 der Meßgasteilstrom

608/610/80                           25

durch die Abzweigleitung 37 außerhalb der Meßvorrichtung geführt ist, wird beim Ausführungsbeispiel gemäß Fig. 4 der Meßgasteilstrom im Innenraum des Festelektrolyten 16 geführt. Insbesondere wird der Meßgasteilstrom durch den Elektrodenraum 23, der an die zweite Elektrode 22 angrenzt, hindurchgeleitet.

Wie aus Figur 4 deutlich zu sehen ist, besitzt der Festelektrolyt 16 an seinem kuppenförmigen geschlossenen Ende eine zentrale, axial verlaufende Durchtrittsöffnung 51, die den Raum 28 und somit den Strömungskanal 29 mit dem Elektrodenraum 23 verbindet. Da der Elektrodenraum 23 durch die Anschlußbohrung 48 stromabwärts der ersten Elektrode 18 wieder an den Meßgaskanal angeschlossen ist, ist somit ein innerer Bypass für den Meßgasteilstrom geschaffen.

Die Durchtrittsöffnung 51 ist hierbei als Drosselstelle ausgebildet und kann auf einfache Weise mit Hilfe eines Absperrkörpers oder vorzugsweise mit dem elektrischen Heizstab 114 in ihrem Querschnitt verändert werden. Hierzu ist der Heizstab oder Heizkörper 114, der zwischen sich und der zweiten Elektrode 22 einen Ringraum 53 freiläßt, mit einem Führungsstab 55 versehen, der mit dem Heizstab 114 fluchtet. Dieser Führungsstab 55 ist mit Hilfe einer nicht dargestellten Stopfbüchse gasdicht durch den Grundkörper 12 in den Außenraum geführt und ermöglicht eine axiale Verstellung des Heizstabes 114. Hierbei wird das kegelförmige Ende des Heizstabes in Richtung auf die ebenfalls kegelförmige Durchtrittsöffnung 51 bewegt und somit die Größe des Durchtritts-

0047434

querschnittes verändert. Gegebenenfalls kann auch ein dichtes Abschließen des Durchtrittsquerschnittes beim vollständigen Hineinschieben des Heizstabes 114 erreicht werden. Auch kann anstelle des Führungsstabes der Heizkörper 114 bis in den Außenraum 82 verlängert werden. Der Einfluß dieses inneren Bypasses auf.die Kennlinie entspricht den Ausführungen gemäß Figur 1.

Ein weiterer Vorteil der Ausführungsform gemäß Figur 4 besteht darin, daß auf einfache Weise die erste oder zweite Elektrode als Meß-Elektrode eingesetzt werden kann, indem durch den Polschalter 21 die Polarität der Elektroden umgeschaltet wird. Bei einem fest eingestellten Durchflußverhältnis zwischen der Strömung im Raum 28; das heißt, im Meßgaskanal, und dem Bypass, das heißt dem Elektrodenraum 23, kann jetzt lediglich durch Betätigen des Polschalters 21 die Empfindlichkeit beziehungsweise der Meßbereich der Meßvorrichtung verändert werden. Setzt man voraus, daß die Größe der Gasströme im Raum 28 und dem Elektrodenraum 23 verschieden ist, so steht die jeweils als Meßelektrode eingesetzte Elektrode nach Art der Betätigung des Polumschalters mit einem Gasstrom anderer Größe in Berührung; somit ist das erfindungsgemäße Meßprinzip erfüllt.

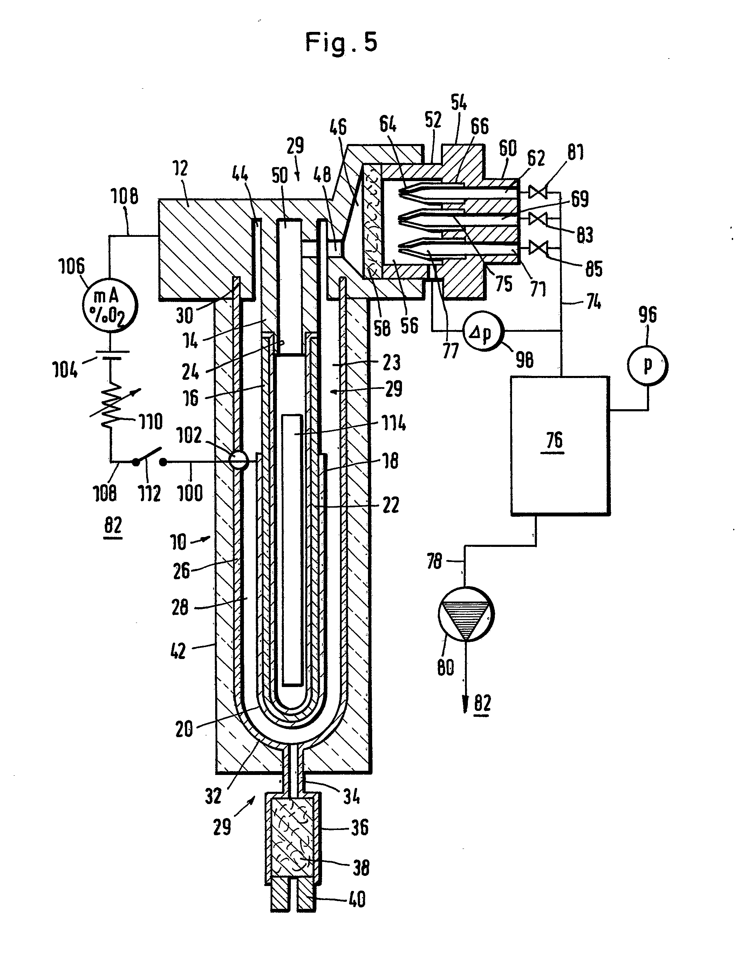

In Figur 5 ist eine Ausführungsvariante dargestellt, bei der in den Meßgasstrom drei als Strömungskonstanthalter dienende Düsen 64, 75 und 77 eingebaut sind. Die drei Düsen 64, 75 und 77 sind zueinander parallel geschaltet. Eine Abzweigleitung 37 mit entsprechenden Drossel- und Absperrorganen, wie sie in

608/610/80                            27

Figur 1 dargestellt ist, weist diese Ausführungsvariante
nicht auf. Im übrigen ist diese Ausführungsform im wesentlichen Baugleich mit der in Figur 1 dargestellten und in der
dazugehörigen Beschreibung erläuterten Ausführungsform. Am
Kopf 54 der Sauerstoffmeßvorrichtung gemäß Figur 5 sind nach
außen ragende Anschlußnippel angeordnet, die jeweils von
einem zur Ausnehmung 56 des Anschlußnippels führenden Kanal
62, 69 und 71 durchdrungen sind. Im Bereich der inneren
Ausmündung weisen die Kanäle 62, 69 und 71 je eine Stufe auf,
in welchen die Düsen 64, 75 und 77 mit ihren jeweils zylindrischen Bereichen befestigt sind, wobei die Düsen frei in
die Ausnehmung 56 ragen. Die Düsen 64, 75 und 77 bilden die
Verengungen, die auch als Blenden oder enge Leitungen ausgebildet sein können. Die hier verwendeten Düsen 64, 75 und 77
sind in gleicher Weise wie die in Figur 3 dargestellte und in
der dazugehörigen Beschreibung erläuterte Düse 64 ausgebildet.

Die in Figur 5 dargestellte Ausführungsform weist ebenso wie
die in Figur 1 dargestellte und in der dazugehörigen
Beschreibung erläuterte Ausführungsform eine Saugvorrichtung
80 auf. Diese ist Baugleich mit der Saugvorrichtung 80 gemäß
der in Figur 1 dargestellten Ausführungsform. Die beiden
Elektroden 18 und 22 der Sauerstoffmeßvorrichtung sind
widerum an eine Gleichspannungsquelle angeschlossen. Mit
Hilfe eines Strommeßgerätes 106, zum Beispiel einem Milliamperemeter wird das dem Sauerstoffgehalt des Meßgases
entsprechende Stromsignal gemessen. Im Inneren des Festelektrolyten 16 ist auch hierbei eine elektrische Heizung 114

608/610/80                                    28

angeordnet, die den Festelektrolyten 16 auf Betriebstemperatur hält.

Bei der in Figur 5 dargestellten Ausführungsform der Sauerstoffmeßvorrichtung kann der Meßgasstrom auf einfache Weise über seine Nenngröße hinaus auf konstante beliebige Werte vergrößert werden. Die Düse 64 ist hierbei so ausgelegt, daß sie für die Konstanthaltung des Nenn-Meßgasstroms verwendet werden kann. Die beiden zusätzlichen Düsen 75 und 77 sind, wie bereits oben erwähnt zu der Düses 64 parallel geschaltet. Die Düsen 75 und 77 sind hierbei bezüglich ihres Durchlaßquerschnittes so ausgelegt, daß die gewünschte Vergrößerung des Meßgasstroms bei ihrem Einschalten in den Gasweg auftritt. Diese zusätzlichen Düsen 75 und 77 sind über die Kanäle 69 un 71 mit derr Anschlußleitung 74 verbunden, wobei jeweils Absperrorgane 83 beziehungsweise 85 zwischengeschaltet sind. Die Düse 64 kann mit dem Absperrorgan 81 ebenfalls an die Anschlußleitung 74 angeschlossen werden. Ergibt sich bei Nennbetrieb mit der Düse 64, welche den ersten Strömungskonstanthalter bildet, eine Kennlinie gemäß a der Figur 6, so wird bei einer Zuschaltung von weiteren Strömungskonstanthaltern, das heißt von weiteren Düsen 75 und 77 der konstante Gasstrom erhöht, so daß die Steigung der Kennlinie und somit die Empfindlichkeit größer wird, wogegen sich der Meßbereich verkleinert. Eine solche Kennlinie ist in Figur 6 mit f bezeichnet. Durch entsprechende Auswahl der Düsen 75 und 77, welche jeweils den zweiten Strömungskonstanthalter darstellen, kann die gewünschte Empfindlichkeit und/oder der gewünschte Meßbereich erzielt werden. Es leuchtet ein, daß durch wahlweises Zuschalten von einzelnen Düsen 75 und 77 oder kombiniertes Zuschalten die Kennlinie feinfühlig verändert werden kann. Dies gilt insbesondere dann, wenn mehrere Düsen, die als zweite Strömungskonstanthalter dienen, vorgesehen sind.

Sollen mit dieser Meßvorrichtung zum Beispiel die Kennlinien a bis f der Figur 6 eingestellt werden, so wird man nicht die Ausführungsformen gemäß der Figuren 1 beziehungsweise 4 mit der Ausführungsform gemäß 5 kombinieren. In vorteilhafter Weise ist in diesem Fall, eine Meßvorrichtung gemäß den Figuren 1 oder 4 zu benutzen. Hierbei werden der Nenn-Gasstrom und die Größe der Meßelektrode so aufeinander abgestimmt, daß sich im Nennbetrieb die steilste Kennlinie gemäß f einstellt. Durch Verringerung des Meßgasstroms mit Hilfe des inneren und/oder äußeren Bypasses können dann sämtliche Kennlinien a bis e der Figur 6 eingestellt werden. Entsprechendes gilt für die Ausführungsform gemäß Figur 5 mit mehreren Strömungskonstanthaltern.

Soll die in Figur 5 dargestellte Meßvorrichtung so ausgelegt werden, daß bei ihr eine Verkleinerung des linearen Meßbereichs, die durch Temperaturänderungen, Alterung oder Verschmutzung der Elektroden hervorgerufen wird, ausgeglichen werden, so werden vorzugsweise drei Strömungskonstanthalter 64, 75 und 77 in den Meßgasstrom eingebaut. Die drei Strömungskonstanthalter sind zueinander parallelgeschaltet.

In Figur 7 sind die Kennlinien der Sauerstoffmeßvorrichtung

608/610/80                          30

mit den drei oben beschriebenen Düsen 64, 75 und 77 dargestellt. Auf der Ordinate ist der Strom in Milliampere aufgezeichnet, welcher für den Transport der Sauerstoffionen von
der ersten Elektrode 18 durch den Festelektrolyten 16 zur
zweiten Elektrode 22 fließt. Auf der Abszisse ist der zugeordnete Sauerstoffgehalt des Meßgases in Volumenprozent
aufgetragen. Im vorliegenden Ausführungsbeispiel soll die
Meßvorrichtung einen Nenn-Meßbereich von 21 Volumenprozent
Sauerstoff aufweisen. Die Größe des Meßgasstromes und die
Größe der ersten Elektrode sind daher so aufeinander abgestimmt, daß innerhalb des Nenn-Meßbereichs ein linearer
Zusammenhang zwischen dem Sauerstoffgehalt und dem elektrischen Strom besteht. Die Kennlinie dieser Meßzelle ist in
Figur 7 mit a bezeichnet. Für die Gewinnung des Eichpunktes
nämlich 21 Volumenprozent Sauerstoff, wozu Außenluft benutzt
wird, sind sätmliche drei Düsen 64, 75 und 77 in den Weg des
Gasstromes zur Konstanthaltung desselben eingeschaltet.

Infolge von Alterungen, Temperaturänderungen und/oder Verschmutzungen der Elektroden 18 und 22 verkleinert sich der
Linearitätsbereich der Meßzelle. Die sich daraus ergebende
Kennlinie ist mit b bezeichnet. Wie aus Figur 7 zu ersehen
ist, besteht ein linearer Zusammenhang zwischen dem fließenden Strom und dem Sauerstoffgehalt bis zu einem Wert von 16
Volumenprozent. Wird jetzt das Absperrorgan 81 geschlossen,
so daß die Düse 64 außer Betrieb genommen wird, so verringert
sich der Meßgasstrom mit dem Erfolg, das die Steigung der
Kennlinie geringer wird und die Meßzelle wieder in der Lage
ist, den gesamten Sauerstoffgehalt des Meßgases zu extrahie-

608/610/80                                    31

ren. Es ergibt sich jetzt die Kennlinie, die mit c bezeichnet ist, und die einen linearen Verlauf aufweist bis zu einem Sauerstoffgehalt von mindestens 21 Volumenprozent hin. Die Linearität im Nenn-Meßbereich ist somit wiederhergestellt. Da sich bei dieser Kennlinienkorrektur die Steigung der Kennlinie und somit die Empfindlichkeit der Meßzelle ändert, ist eine neue Eichung erforderlich, die entsprechend den Ausführungen weiter oben durchgeführt wird.

Ergibt sich nun nach einiger Betriebszeit eine weitere Einschränkung des linearen Meßbereichs, so daß die Meßvorrichtung jetzt die mit d bezeichnete Kennlinie aufweist, so wird das Absperrorgan 83 geschlossen und die Düse 75 ausser Betrieb genommen. Damit wird der Meßgasstrom weiter verringert. Es ergibt sich dann eine Kennlinie e, welche innerhalb des gewünschten Nenn-Meßbereichs linear verläuft. Allerdings ist auch hier die Empfindlichkeit der Meßzelle zurückgegangen und eine neue Eichung erforderlich. Im vorliegenden Ausführungsbeispiel sind die Durchtrittsquerschnitte der drei Düsen 65, 75 und 77 untereinander gleich, so daß die Steigung der Kennlinie a beim Absperren der einzelnen Düsen sich jeweils um ein Drittel verringert, wie deutlich aus Figur 7 zu ersehen. Die Düsen können jedoch auch so ausgelegt werden, daß der Durchfluß der ersten Düse 64 dem halben maximalen Gasstrom entspricht. Die beiden anderen Düsen 75 und 77 weisen gleiche Durchtrittsquerschnitte auf, der gesamte Durchfluß durch die beiden Düsen entspricht dann dem halben maximalen Gasstrom.

608/610/80                        32

Bei einer Vielzahl von Düsen, die in beliebiger Kombination absperrbar sind kann eine feine Abstufung des Durchflusses ermöglicht werden und somit eine weitgehende und feinfühlige Anpassung der Kennlinien durchgeführt werden.

608/610/80                    33

Mannheim                          6. Juli 1981
Mp.-Nr. 608/610/80               ZPT/P1 - Kr/Sd

## Ansprüche

1. Sauerstoffmeßvorrichtung insbesondere für Verbrennungsabgase, mit einem sauerstoffionenleitenden Festelektrolyten (16) der mit wenigstens zwei Elektroden (18, 22) versehen ist, von denen eine erste Elektrode (18) als Meß-Elektrode eingesetzt und an einen Meßgaskanal (29) angrenzt, der einen ersten Strömungskonstanthalter (64) aufweist und mit einer Meßgasquelle verbindbar ist, wobei beide Elektroden (18, 22) unter Einschaltung eines Strommeßgerätes (106) mit einer elektrischen Gleichspannungsquelle (104) verbindbar sind, dadurch gekennzeichnet, daß wenigstens ein weiterer auf einen festen Durchlaßwert eingestellter zweiter Strömungskonstanthalter (75, 77) angeordnet dem ersten Strömungskonstanthalter (64) parallel geschaltet ist.

2. Sauerstoffmeßvorrichtung nach Anspruch 1, dadurch gekennzeichnet, daß an den Meßgaskanal (29) stromaufwärts der ersten Meßelektrode (18) eine Abzweigleitung (37) für die

608/610/80                                    34

Abfuhr eines Meßgasteilstromes angeschlossen ist.

3. Sauerstoffmeßvorrichtung, insbesondere für Verbrennungsabgase, mit einem sauerstoffionenleitenden Festelektrolyten (16), der mit wenigstens zwei Elektroden (18, 22)
versehen ist, von denen eine erste Elektrode (18) als Meßelektrode eingesetzt und an einen Meßgaskanal (29) grenzt,
der einen ersten Strömungskonstanthalter (64) aufweist und
mit einer Meßgasquelle verbindbar ist, wobei beide Elektroden
unter Einschaltung eines Strommeßgerätes (106) mit einer
elektrischen Gleichspannungsquelle (104) verbindbar sind,
dadurch gekennzeichnet, daß im Meßgaskanal (29) wenigstens
ein weiterer auf einen festen Durchlaßwert einstellbarer
Strömungskonstanthalter (75, 77) angeordnet und dem ersten
Strömungskonstanthalter (64) parallel geschaltet ist, oder
daß an den Meßgaskanal (29) stromaufwärts der ersten Meßelektrode (18) eine Abzweigleitung (37) für die Abfuhr eines
Meßgasteilstromes angeschlossen ist.

4. Sauerstoffmeßvorrichtung nach Anspruch 1, dadurch
gekennzeichnet, daß im Wege des Meßgasstromes drei als
Strömungskonstanthalter (64, 75 und 77) dienende Verengungen
angeordnet sind, deren Durchtrittsquerschnitte so gewählt
sind, daß die Verengungen (64, 75 und 77) in Parallelschaltung einen ausreichenden maximalen Gasstrom durchlassen.

5. Sauerstoffmeßvorrichtung nach Anspruch 4, dadurch
gekennzeichnet, daß die Größe des maximalen Meßgasstromes auf
die Größe der ersten Elektrode (18) im Neuzustand der Meß-

608/610/80                              35

zelle abgestimmt ist, und daß die Verengungen (64, 75 und 77)
wahlweise einzeln, in Parallelschaltung gemeinsam oder
beliebig kombiniert in den Weg des Meßgasstromes einschaltbar
sind.

6. Sauerstoffmeßvorrichtung nach Anspruch 4 bis 5,
dadurch gekennzeichnet, daß drei als Düsen ausgebildete
Verengungen (64, 75 und 77) vorgesehen sind.

7. Sauerstoffmeßvorrichtung nach Anspruch 4 bis 6,
dadurch gekennzeichnet, daß die drei Verengungen (64, 75 und
77) alle den gleichen Durchtrittsquerschnitt aufweisen.

8. Sauerstoffmeßvorrichtung nach Anspruch 4 bis 6,
dadurch gekennzeichnet, daß die erste Verengung (64) für den
Durchlaß des halben maximalen Gasstromes ausgebildet ist,
während die beiden  Verengungen (75 und 77) untereinander
gleiche Durchtrittsquerschnitte und zusammen ebenfalls für
den Durchfluß des halben maximalen Meßgasstromes ausgelegt
sind.

9. Sauerstoffmeßvorrichtung nach Anspruch 1 bis 8,
dadurch gekennzeichnet, daß der Meßgasstrom mit Hilfe der
Strömungskonstanthalter (64, 75 und 77) auf konstante, von
der Nenngröße abweichende Werte einstellbar ist.

10. Sauerstoffmeßvorrichtung nach Anspruch 1 bis 9,
dadurch gekennzeichnet, daß der Meßgasstrom zur Erweiterung
des Nennmeßbereichs und/oder zur Verringerung der

608/610/80                                    36

Nennempfindlichkeit durch Verschließen einer oder mehrerer Strömungskonstanthalter (64, 75 und 77) verminderbar ist.

11. Sauerstoffmeßvorrichtung nach Anspruch 1 bis 9, dadurch gekennzeichnet, daß der Meßgasstrom zur Verkleinerung des Nennmeßbereichs und/oder zur Steigerung der Nennempfindlichkeit durch die Zuschaltung einer oder mehrerer Strömungskonstanthalter (64, 75 und 77) vergrößerbar ist.

12. Sauerstoffmeßvorrichtung nach Anspruch 1 bis 11, dadurch gekennzeichnet, daß der zweite und der dritte Strömungskonstanthalter (75 und 77) jeweils eine Verengung mit einem bei Schallgeschwindigkeit des Meßgases einen den gewünschten zusätzlichen Meßgasstrom durchlassenden Durchtrittsquerschnitt aufweisen, und daß zwischen Abströmseite und Zuströmseite der Verengung (75 und 77) ein Druckverhältnis einstellbar ist, das in der Verengung die Schallgeschwindigkeit des Meßgasstromes bewirkt (Figur 5).

13. Sauerstoffmeßvorrichtung nach Anspruch 1 bis 3, dadurch gekennzeichnet, daß in der Abzweigleitung (37) ein vorzugsweise einstellbares Drossel- beziehungsweise Absperrorgan (39) vorgesehen ist.

14. Sauerstoffmeßvorrichtung nach Anspruch 1 bis 3, dadurch gekennzeichnet, daß bei Überdruck des Meßgases und einem von der Abzweigleitung (37) in den Meßgaskanal (29) eingeschalteten Strömungskonstanthalter die Abzweigleitung (37) in den Außenraum (82) mündet.

15. Sauerstoffmeßvorrichtung nach Anspruch 1 bis 3, dadurch gekennzeichnet, daß bei einem stromabwärts der ersten Elektrode (18) im Meßgaskanal (29) angeordneten Strömungskonstanthalter (64) das Ende der Abzweigleitung (37) stromabwärts der ersten Elektrode (18) vor dem ersten Strömungs- konstanthalter (64) àn den Meßgaskanal (29) angeschlossen ist (Figur 1).

16. Sauerstoffmeßvorrichtung nach Anspruch 1 bis 3, dadurch gekennzeichnet, daß die Abzweigleitung (37) mehrere parallel geschaltete, wahlweise einschaltbare Teilleitungen (41) aufweist (Figur 1).

17. Sauerstoffmeßvorrichtung nach einem der Ansprüche 1 bis 3, dadurch gekennzeichnet, daß bei einem Festelektrolyten (16) der den Meßgaskanal (29) von einem vorzugsweise an die zweite Elektrode (22) grenzenden Elektrodenraum (23) trennt, wobei dieser stromaufwärts der ersten Elektrode (18) an den Meßgaskanal (29) angeschlossen ist, die Abzweigleitung von mindestens einer Durchtrittsöffnung (51) des Festelektrolyten (16) und dem Elektrodenraum (23) mitgebildet ist (Figur 4).

18. Sauerstoffmeßvorrichtung nach Anspruch 17, dadurch gekennzeichnet, daß die Durchtrittsöffnung (51) als Drosselstelle ausgebildet ist.

19. Sauerstoffmeßvorrichtung nach Anspruch 17 und 18, dadurch gekennzeichnet, daß bei einem rohrförmigen, an einem

608/610/80     38

Ende geschlossenen Festelektrolyten (16) der im Inneren einen Elektrodenraum (23) aufweist, die Durchtrittsöffnung (51) am geschlossenen Ende des Festelektrolyten (16) angeordnet und in ihrem Querschnitt durch einen axial verschiebbaren Absperrkörper (113) veränderbar ist (Figur 4).

20. Sauerstoffmeßvorrichtung nach Anspruch 19, dadurch gekennzeichnet, daß die Durchtrittsöffnung (51) sich in Strömungsrichtung kegelförmig erweitert und die Spitze des Absperrorgans (113) einen entsprechenden Gegenkegel aufweist.

21. Sauerstoffmeßvorrichtung nach Anspruch 17 bis 20, dadurch gekennzeichnet, daß zum wahlweisen Einsatz der ersten oder zweiten Elektrode als Meßelektrode die elektrische Gleichspannungsquelle (104) über einen Polumschalter (21) in den elektrischen Stromkreis einschaltbar ist.

22. Sauerstoffmeßvorrichtung nach Anspruch 19 bis 21, dadurch gekennzeichnet, daß der Absperrkörper (113) als Heizkörper (114) ausgebildet und sich vorzugsweise über den gesamten Bereich der Elektroden (18 und 22) erstreckt.

1/4

Fig.1

Fig.2

Fig.3

Fig.4

Fig. 5

4/4

Fig.6

[mA]

Fig.7

[mA]

Europäisches
Patentamt

**EUROPÄISCHER RECHERCHENBERICHT**

Nummer der Anmeldung

EP 81 10 6629

| | **EINSCHLÄGIGE DOKUMENTE** | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| E,P | EP - A - 0 028 826 (BROWN, BOVERI & CIE.)<br><br>* Zusammenfassung *<br><br>-- | 1 | G 01 N 33/00<br>G 01 N 27/56 |
| A | US - A - 4 128 458 (J.O.OBIAYA)<br><br>* Zusammenfassung; Spalte 1, Spalte 2, Zeilen 1-32 *<br><br>-- | 1 | |
| A | FR - A - 2 115 080 (VEB KOMBINAT MESS- UND REGELUNGSTECHNIK)<br><br>* Figur 13, Seite 16, Zeilen 30-40; Seite 17 *<br><br>-- | 1 | **RECHERCHIERTE SACHGEBIETE (Int. Cl.³)**<br><br>G 01 N 33/00<br>G 01 N 27/56 |
| A | FR - A - 2 297 411 (BAILEY METER CO.)<br><br>* Ansprüche *<br><br>-- | 1 | |
| A | DE - A - 2 338 873 (R.BOSCH)<br><br>* Figur 4; Ansprüche *<br><br>-- | 1 | |
| A | FR - A - 2 398 301 (R.BOSCH)<br><br>* Figur 2, Ansprüche *<br><br>---------- | 22 | **KATEGORIE DER GENANNTEN DOKUMENTE**<br><br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 11-12-1981 | CALLEWAERT-HAEZ |

EPA form 1503.1   06.78